# FASCICULE DE BREVET EUROPEEN

(11) **EP 1 639 988 B1**
(45) Date de publication et mention de la délivrance du brevet: **17.06.2009**
(21) Numéro de dépôt: 05291366.2
(22) Date de dépôt: 24.06.2005
(51) Int. Cl.: A61K 8/04, A61K 8/92, A61K 8/896, A61Q 1/00, A61Q 1/02, A61Q 1/06, A61Q 1/08, A61Q 1/10

(54) **Emulsion cosmétique eau-dans-huile solide**
Kosmetische feste W/Ö Emulsion
Cosmetic solid W/O emulsion

(30) Priorité: 16.07.2004 FR 0451550; 16.07.2004 FR 0451548
(43) Date de publication de la demande: 29.03.2006
(73) Titulaire: L'ORÉAL, 75008 Paris (FR)
(72) Inventeur: Themens, Agnès, 92340 Bourg la Reine (FR)
(74) Mandataire: Boulard, Denis

(56) Documents cités:
- EP-A- 1 036 553
- EP-A- 1 068 852
- EP-A- 1 416 016
- EP-A- 1 473 017
- EP-A- 1 481 660
- WO-A-99/47111
- WO-A-03/000223
- WO-A-03/028690
- DE-A1- 10 162 841
- PATENT ABSTRACTS OF JAPAN vol. 017, no. 391 (C-1087), 22 juillet 1993 (1993-07-22) & JP 05 070337 A (KOSE CORP), 23 mars 1993 (1993-03-23)
- PATENT ABSTRACTS OF JAPAN vol. 2000, no. 15, 6 avril 2001 (2001-04-06) & JP 2000 351712 A (KOSE CORP), 19 décembre 2000 (2000-12-19)

## Description

La présente invention a pour objet une émulsion solide eau-dans-huile comprenant une cire et un tensioactif siliconé particulier, utilisable dans le domaine cosmétique. L'invention a également pour objet un procédé de maquillage ou de soin de la peau d'être humain comprenant l'application de la composition sur la peau.

La composition de maquillage selon l'invention est en particulier une composition de maquillage de la peau, telle qu'un fond de teint, un fard à paupières, un fard à joue, un produit anticernes, un produit de maquillage du corps, un rouge à lèvres. Plus spécialement, l'invention porte sur une composition de fond de teint.

La composition de soin peut être un produit de soin de la peau tels qu'une base de soin pou la peau, une crème de soin (crème de jour, de nuit, anti-rides), une base de maquillage ; une composition de soin pour les lèvres (baume à lèvres) ; une composition de protection solaire ou autobronzante ; un déodorant.

Les produits de maquillage de la peau comme les fonds de teint sont connus sous formes de galéniques très diverses : poudre libre, poudre compacte, produit solide coulé, stick, crème fluide.

Les produits solides coulés peuvent être anhydres ou bien sous forme d'émulsions. Ces dernières contiennent généralement des corps gras tels que des huiles et des cires solides, de l'eau et une phase particulaire généralement composée de charges et de pigments.
L' émulsion solide ne s'écoule pas sous son propre poids à la température ambiante et est adaptée pour être conditionnée dans un boîtier : pour appliquer le produit, l'utilisatrice peut prendre directement le produit en le délitant à l'aide des doigts ou d'un applicateur tel qu'une éponge. Pour une bonne prise du produit par l'utilisatrice, il est nécessaire que l'émulsion solide présente une dureté satisfaisante et notamment que cette dureté varie peu au cours du temps, notamment après un stockage à des températures plus élevée que la température ambiante (25 °C). Or certaines émulsions solides peuvent présenter une chute de leur dureté au cours du temps : le produit a alors tendance à ce ramollir, rendant la prise de celui-ci plus difficile, voire désagréable pour l'utilisatrice qui peut difficilement contrôler la quantité de produit prélevé lors de la prise. De plus, un produit initialement solide et qui se ramollit n'est pas attrayant pour l'utilisatrice.

EP-A-1 481 660 (SHIN-ETSU CHEMICAL CO., LTD) décrit (ex. 55) une émulsion solide eau-dans-huile comprenant une phase aqueuse émulsionnée dans une phase grasse comprenant une huile ester (cetyl isooctanoate) et une cire (paraffin wax), et un tensioactif siliconé à chaîne polyhydroxylée (KF6104).

Le but de la présente invention est donc de disposer d'une composition solide de maquillage ou de soin de la peau présentant une dureté stable dans le temps, notamment après un stockage d'au moins un mois à 37 °C.

Un autre but de l'invention est de disposer d'une composition solide présentant une bonne dureté tout en ayant une texture crémeuse et onctueuse lors de l'application sur la peau.

Les inventeurs ont découvert qu'en utilisant une huile ester, une cire, un tensioactif émulsionnant particulier, il est possible d'obtenir une émulsion solide présentant une dureté stable dans le temps, notamment après 1 mois à 37 °C, et ayant également une texture onctueuse et crémeuse lors de son application sur la peau.

De façon plus précise, l'invention a pour objet une émulsion solide eau-dans-huile comprenant une phase aqueuse émulsionnée dans une phase grasse comprenant une huile ester et une cire de Candelilla et d'ozokérite, un tensioactif alkyl C₈-C₂₂ diméthicone copolyol, et un tensioactif siliconé à chaîne polyhydroxylée.

L'invention a également pour objet un procédé cosmétique de maquillage ou de soin non thérapeutique de la peau comprenant l'application sur la peau d'une composition telle que définie précédemment.

On entend par composition solide une composition qui ne s'écoule pas sous son propre poids à température ambiante (25 °C) au bout d'une heure.

L'émulsion solide selon l'invention comprend au moins une huile ester.

Par huile ester, on entend une huile liquide à la température ambiante (25 °C) comprenant au moins une fonction ester dans sa molécule. Avantageusement, l'huile ester est un monoester.

L'huile ester peut être choisie de préférence parmi les monoesters de formule R₁COOR₂ dans laquelle R₁ représente une chaîne hydrocarbonée, linéaire ou ramifiée, comportant de 4 à 40 atomes de carbone, de préférence de 4 à 30 atomes de carbone, et préférentiellement de 7 à 20 atomes de carbone, et R₂ représente une chaîne hydrocarbonée ramifiée contenant de 3 à 40 atomes de carbone, de préférence de 10 à 30 atomes de carbone, et préférentiellement de 16 à 26 atomes de carbone .

Comme exemple d'huile ester, on peut citer le néopentanoate d'isodécyle ; l'octanoate d'isocétyle ; l'isononanoate d'isononyle, l'isononanoate d'isodécyle, l'isononanoate de tridécyle ; le laurate d'hexyle, le laurate de 2-hexyl-décyle ; le myristate d'isopropyle, le myristate d'isocétyle, le myristate d'isotridécyle le myristate de 2-octyldodécyle ; le palmitate d'isopropyle, le palmitate de 2-éthyl-hexyle, le palmitate d'isooctyle, le palmitate d'isocétyle, le palmitate d'isodécyle le palmitate d'isostéaryle, le palmitate de 2-octyl-décyle ; l'isostéarate d'isopropyle, le stéarate d'octyl-2 dodécyle, l'isostéarate d'isostéaryle ; l'érucate d'octyl-2 dodécyle ; et leurs mélanges.

L'huile ester peut être présente dans l'émulsion selon l'invention en une teneur allant de 5 % à 20 % en poids, par rapport au poids total de la composition, de préférence allant de 7 % à 15 % en poids, et préférentiellement allant de 8 % à 12 % en poids.

La phase grasse peut comprendre également une huile de silicone volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

L'huile de silicone volatile peut être choisie parmi les huiles de silicones linéaires ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

L'huile de silicone volatile peut être présente en une teneur allant de 5 % à 40 % en poids, par rapport au poids total de la composition, de préférence allant de 10 % à 30 % en poids, et préférentiellement allant de 15 % à 30 % en poids.

Avantageusement, la teneur totale en eau et en huile de silicone volatile dans la composition est inférieure ou égale à 50 % en poids, par rapport au poids total de la composition, notamment va de 30 % à 50 % en poids, et de préférence va de 40 % à 50 % en poids.

La phase grasse de l'émulsion peut comprendre une huile additionnelle, différente des huiles esters et des huiles de silicone volatiles décrites précédemment.

L'huile additionnelle peut être notamment choisies parmi les huiles hydrocarbonées ou les huiles fluorées, volatiles ou non volatiles.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, éther, acide carboxylique, amine et/ou amide.

Par huile fluorée, on entend une huile contenant au moins un atome de fluor.

Comme huile additionnelle volatile utilisable dans l'invention, on peut citer :
- les huiles volatiles hydrocarbonées ayant de 8 à 16 atomes de carbone et leurs mélanges et notamment les alcanes ramifiés en C₈-C₁₆ comme les isoalcanes (appelées aussi isoparaffines) en C₈-C₁₆, l'isododécane, l'isodécane, l'isohexadécane et par exemple les huiles vendues sous les noms commerciaux d'Isopars' ou de Permethyls', les esters ramifiés en C₈-C₁₆ comme le néo pentanoate d'isohexyle et leurs mélanges ; on utilise de préférence l'isododécane ;
- les huiles fluorées volatiles telles que le nonafluoroéthoxybutane, le nonafluoro-méthoxybutane, le décafluoropentane, le tétradécafluorohexane, le dodécafluoropentane, et leurs mélanges.
- et leurs mélanges.

Comme huile additionnelle non volatile utilisable dans l'invention, on peut citer les huiles hydrocarbonées d'origine minérale ou synthétique telles les hydrocarbures linéaires ou ramifiés comme l'huile de paraffine ou ses dérivés, l'huile de vaseline, les polydécènes, le polyisobutène hydrogéné tel que le Parléam commercialisé par la société Nippon Oil Fats, le squalane d'origine synthétique ou végétale ;

Comme huile additionnelle non volatile, on peut également citer les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, notamment les triglycérides d'acide gras notamment de 4 à 22 atomes de carbone, comme les triglycérides des acides heptanoïque, octanoïque, et des acides caprique/caprylique ou bien encore les triglycérides hydroxylés, telles que l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive, de germes de céréales (maïs, blé, orge, seigle), de beurre de karité ;
les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alcoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ;
et leurs mélanges.

Avantageusement, l'huile additionnelle peut être présente en une teneur allant de 0,1 % à 10 % en poids, par rapport au poids total de la composition, et de préférence allant de 0,1 % à 5 % en poids. Dans un mode de réalisation de l'émulsion selon l'invention, la teneur en huile additionnelle peut aussi être de 0 % en poids, par rapport au poids total de la composition.

Selon un mode préféré de réalisation de l'invention, l'émulsion contient comme huile uniquement une huile ester et éventuellement une huile de silicone volatile, telles que décrites précédemment.

Avantageusement, la cire présente dans l'émulsion selon l'invention peut être choisie parmi les cires ayant une dureté allant de 5MPa à 9 MPa, et de préférence allant de 6 MPa à 9 MPa, et préférentiellement allant de 7 MPa à 9 MPa.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

La dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Les cires, au sens de la demande, peuvent être hydrocarbonées, siliconées et/ou fluorées, comportant éventuellement des fonctions ester ou hydroxyle.

La cire ayant la dureté telle que définie précédemment peut être choisie parmi la cire de Carnauba, les cires microcristallines, les ozokérites, l'huile de jojoba hydrogénée, les cires de polyéthylène telles que celle vendue sous les dénominations "PERFORMALENE 400 POLYETHYLENE" par la société New Phase Technologies, les cires de silicone comme les poly alkyle C24-C28 méthyl dimethyl siloxane telle que celle vendue sous la dénomination "ABIL WAX 9810" par la société GOLDSCHMIDT, le beurre de palme, le stéarate d'alkyle en C20-C40 vendu sous la dénomination "KESTER WAX K82H" par la société Kester Keunen, le benzoate de stéaryle, la cire de Shellac, et leurs mélanges. On utilise de préférence une cire choisie parmi la cire de Carnauba, la cire de Candelilla, les ozokérites, l'huile de jojoba hydrogénée, les cires de polyéthylène. La cire est choisie parmi les mélanges de cire de Candelilla et d'ozokérite.

La cire peut être présente dans la composition selon l'invention en une teneur allant de 1 % à 10 % en poids, par rapport au poids total de la composition, de préférence allant de 2 % à 7 % en poids, et préférentiellement allant de 2 % à 5 % en poids.

L'émulsion selon l'invention comprend un tensioactif siliconé à chaîne polyhydroxylée pouvant être choisi parmi les polymères de silicones à chaîne polyhydroxylée ou les élastomères de silicone à chaîne polyhydroxylée. On entend par chaîne polyhydroxylée un chaîne hydrocarbonée comprenant au moins deux groupements hydroxyles.

On peut notamment utiliser les polymères de silicone représentés par la formule générale (I') suivante :

R¹ₐ R²_{b} R³_{c} SiO_{(4-a-b-c)/2} (I')

dans laquelle :
a) a, b et c sont tels que a varie de 1 à 2,5 ; et b et c, indépendamment l'un de l'autre, varient de 0,001 à 1,5.
b) R¹, identique ou différent, est choisi parmi :
   - les radicaux alkyle en C₁ à C₃₀, le cas échéant substitués par un ou plusieurs atomes de fluor, groupements amino et/ou carboxy,
   - les radicaux aryle, aralkyle, et
   - les radicaux de formule générale (II) :

      ― C_{d}H_{2d}-O-(C₂H₄O)ₑ(C₃H₆O)_{f}R⁴ (II)

      dans laquelle :
      - R⁴ étant un radical hydrocarboné en C₄ à C₃₀, ou un radical R⁵-(CO)- avec R⁵ étant un radical hydrocarboné en C₁ à C₃₀, et
      - d, e et f étant des entiers tels que d varie de 0 à 15, e et f, indépendamment l'un de l'autre, varient de 0 à 50, et
   - leurs combinaisons.
c) R² est réprésenté par la formule générale suivante (III) :

   -Q-O-X (III)

   dans laquelle :
   - Q est un radical hydrocarboné bivalent en C₂ à C₂₀, pouvant inclure au moins une liaison éther et/ou au moins une liaison ester, et
   - X est un radical hydrocarboné polyhydroxylé.
d) R³ est un groupement organosiloxane de formule générale (IV) : 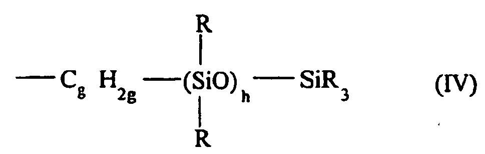 avec :

- chacun des radicaux R représentant, indépendamment l'un de l'autre, un radical choisi parmi les radicaux alkyle en C₁ à C₃₀, le cas échéant substitué par un ou plusieurs atomes de fluor, et les radicaux aryle et aralkyle,
- g et h étant des entiers tels que g varie de 1 à 5 et h varie de 0 à 500.

Lorsque les radicaux R représentent un radical choisi parmi les radicaux alkyle en C₁ à C₃₀, le cas échéant substitués par un ou plusieurs atomes de fluor, les radicaux aryle et les radicaux aralkyle, ils ont la même signification que le radical R¹ tel que défini précédemment.

Il est à noter que les radicaux R¹, R² et R³ des polymères de silicone de formule générale (I'), telle que définie ci-dessus, ne sont pas distribués de manière séquentielle, mais de manière aléatoire ou statistique, c'est-à-dire qu'ils apparaissent dans la structure du polymère sans ordre déterminé.

Dans a) :
- de manière plus particulière, a varie de 1,2 à 2,3. Et, en particulier, b et c, indépendamment l'un de l'autre, varient de 0,05 à 1.

Dans b) :
- lorsque R¹ est un radical alkyle, il peut être un radical alkyle en C₁ à C₃₀, en particulier un radical alkyle en C₁ à C₂₅, plus particulièrement un radical alkyle en C₁ à C₂₀, en particulier un radical alkyle en C₁ à C₁₀, et notamment un radical alkyle en C₁ à C₆, et en particulier un radical alkyle en C₁ à C₄. Plus particulièrement, il peut être un radical méthyle, éthyle, n- ou iso-propyle, n- ou iso- ou tert- butyle, pentyle, hexyle, heptyle, octyle, nonyle, décyle, undécyle ou lauryle. Il peut également être un radical cycloalkyle tel qu'un cyclopropyle, un cyclobutyle, cyclopentyle ou un cyclohexyle. Il peut également être un radical alkyle monoinsaturé ou polyinsaturé, linéaire ou ramifié. Il peut également être un radical alkyle substitué par un ou plusieurs atomes de fluor, tel que le trifluoropropyle ou l'heptadécafluorodécyle. Il peut également être un radical alkyle substitué par un ou plusieurs groupements amino, tels que l'amino-2 éthyle, l'amino-3 propyle, le (amino-2 éthyle)amino-3 propyle. Il peut également être un groupe alkyle substitué par un ou plusieurs groupements carboxy, tel que le carboxy-3 propyle.
- R¹ peut également être un radical aryle ou aralkyle tel que le radical phényle, le radical tolyle, le radical benzyle et le radical phénéthyle.
- R¹ peut également être un groupe organique représenté par la formule générale (II) :

   ― C_{d}H_{2d}-O-(C₂H₄O)ₑ(C₃H₆O)_{f}R⁴ (II)

Selon un mode de réalisation particulier, R¹ peut être un radical hydroxylé ou un radical issu de la réaction d'addition d'un alkényléther, saturé ou insaturé, ramifié ou linéaire, dans lequel d = 0 et donc de formule :

-O-(C₂H₄O)ₑ(C₃H₆O)_{f}R⁴

Dans ce cas, lorsque e et f égalent zéro, alors R¹ est un groupe alcoxy ayant de 4 à 30 atomes de carbone, par exemple un radical alcoxy inférieur en C₄ à C₁₀, tel que le butoxy ou le pentoxy ou un radical alcoxy supérieur, en C₁₁ à C₃₀, tel que l'oléoxy, le stéaroxy, à savoir par exemple, l'alcool cétylique, l'alcool oléique et l'alcool stéarylique, ou un radical issu d'un acide ou d'un acide gras, tel que l'acide acétique, l'acide lactique, l'acide butyrique, l'acide oléique, l'acide stéarique et l'acide béhénique.

Lorsque e et f sont supérieurs à 1, alors R¹ est un radical hydroxyle provenant de la réaction d'addition d'un alkylène oxyde.

Lorsque e et f égalent zéro, il est particulièrement avantageux que d soit égal à 3, 5 ou 11. Dans ce cas, R¹, selon la nature du substituant R⁴, est un radical allyléther, penthényléther ou undécényléther, ou un radical allylstéaryléther, penthénylbéhényléther, ou undécényloléyléther.

Lorsque e ou f sont différents de zéro, un radical alcoxy et un radical ester sont présents *via* un groupement polyoxyalkylène.

Quels que soient e et f, il est particulièrement avantageux que d soit compris dans l'intervalle variant de 3 à 5.

Selon un mode de réalisation, le radical R¹ peut être l'un quelconque des radicaux précédemment définis, ou une combinaison de deux ou plusieurs de ces radicaux.

De manière avantageuse, R¹ est un radical alkyle choisi parmi le radical méthyle, le radical lauryle, et leurs combinaisons.

Par ailleurs, lorsque R¹ correspond à deux ou plusieurs radicaux, par exemple un radical méthyle et un radical lauryle, ceux-ci apparaissent dans la structure de manière aléatoire, et avec une fréquence susceptible d'être différente pour chacun des radicaux.

De manière particulière, au moins 50 % des radicaux R¹, en particulier au moins 70 % des radicaux R¹, et plus particulièrement 100 % des radicaux R¹ sont des radicaux méthyle.

Dans c) :
- Q peut être en particulier un radical hydrocarboné bivalent choisi parmi:
   -(CH₂)₂-,-(CH₂)₃-, CH₂CH(CH₃) CH₂, - (CH₂)₄-, -(CH₂)₅-, -(CH₂)₆-, - (CH₂)₇- (CH₂)₈-, -(CH₂)₂-CH(CH₂CH₂CH₃)-, -CH₂-CH(CH₂CH₃)-, -(CH₂)₃-O-(CH₂)₂-, -(CH₂)₃-O-(CH₂)₂-O-(CH₂)₂-, -(CH₂)₃-O-CH₂CH(CH₃)- et -CH₂-CH(CH₃)-COO (CH₂)₂-.

De manière avantageuse, Q est un radical bivalent choisi parmi - (CH₂)₂- et -(CH₂)₃-.
- X peut être de manière particulière un radical hydrocarboné polyhydroxylé comprenant au moins deux résidus hydroxyles, et en particulier un groupement hydrocarboné choisi parmi les dérivés glycérilés et les dérivés osidiques.

Les résidus glycérols peuvent être des composés ayant les formules suivantes, dans lesquelles Q a la même signification que dans la formule générale (III), et s et t sont des entiers compris dans l'intervalle variant de 1 à 20, en particulier de 1 à 15, en particulier de 1 à 10, et plus particulièrement de 1 à 5.

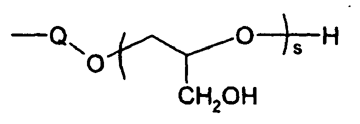

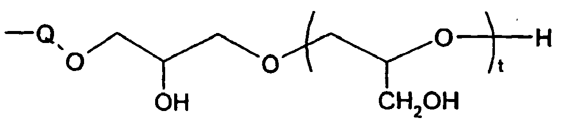

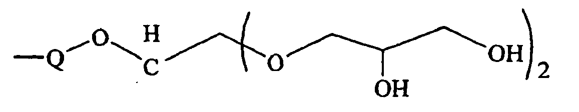

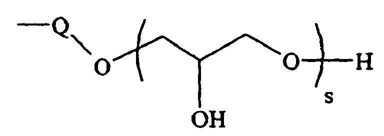

Dans les formules précédentes, un ou plusieurs groupes hydroxyles peuvent être remplacés par des groupes alcoxy ou des groupements esters.

Les radicaux osidiques utilisables dans la formule générale (III) peuvent être de type monosaccharidique, tel que les groupements glycosyle, mannosyle, galactosyle, ribosyle, arabinosyle, xylosyle ou fructosyle, de type oligosaccharidique tel que le maltosyle, le cellobiosyle, le lactosyle ou le maltotriosyle, ou de type polysaccharidique tel que la cellulose ou l'amidon.

De manière particulière, les groupements osidiques sont de type monosaccharidique ou oligosaccharidique.

Dans d) :
- chacun des radicaux R peut représenter en particulier, indépendamment l'un de l'autre, un radical choisi parmi les radicaux alkyle en de C₁ à C₂₀, plus particulièrement en C₁ à C₁₀, en particulier en C₁ à C₆, le cas échéant substitué par un ou plusieurs atomes de fluor. Lorsque les radicaux R représentent un radical choisi parmi les radicaux alkyle tels que définis précédemment, le cas échéant substitués par un ou plusieurs atomes de fluor, ils ont la même signification que le radical R¹ tel que défini précédemment.
- g, selon un mode particulier de réalisation, est égal à 2
- h, selon un mode particulier de réalisation, est compris dans l'intervalle variant de 1 à 50.

Selon un mode particulier de réalisation, le polymère de silicone de formule générale (I') est tel que :
- a varie de 1 à 1,4, b et c, indépendamment l'un de l'autre, varient de 0,02 à 0,03, et
- R¹ est un radical alkyle en C₁ à C₁₀, en particulier en C₁ à C₆, et plus particulièrement en C₁ à C₄.
- R² est représenté par la formule (IIIA) :

   C₃H₆O[CH₂CH(OH)CH₂O]ₙ H (IIIA)

   dans laquelle :
- n varie de 1 à 5, et
- R³ est représenté par la formule (IVA) :

   -C₂H₄(CH₃)₂SiO[(CH₃)₂SiO]ₘ Si(CH₃)₂ (IVA)

   dans laquelle :
- m varie de 3 à 9.

Selon un autre mode particulier de réalisation, le polymère de silicone de formule générale (I'), utilisable dans les compositions cosmétiques selon la présente invention, est tel que :
- a varie de 1 à 1,4, b et c, indépendamment l'un de l'autre, varient de 0,02 à 0,04,
- R¹ est un radical méthyle,
- R² est représenté par la formule (IIIA) dans laquelle n varie de 1 et 5, et
- R³ est représenté par la formule (IVA) dans laquelle m varie de 3 à 9.

Par exemple, le polymère de silicone de formule générale (l') utilisé dans la composition selon l'invention, est choisi parmi la diméthicone de polyglycéryl-3 polyméthylsiloxyéthyle, la diméthicone de laurylpolyglycéryl-3 polyméthylsiloxyéthyle et la diméthicone de polyglycéryl-3 disiloxane, dont les formules sont respectivement :
- Diméthicone de polyglycéryl-3 polyméthylsiloxyéthyle (formule (V)) : 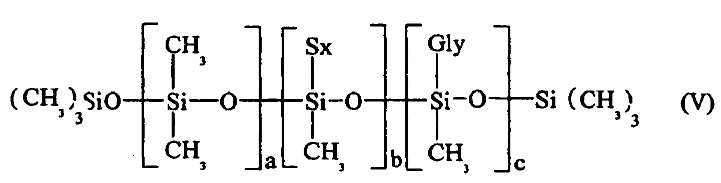
dans laquelle :
Sx : -C₂H₄ [(CH₃)₂SiO]ₘSi(CH₃)₃
Gly : -C₃H₆O[CH₂-CH(OH)CH₂O]ₙH
   avec a = 1-1,4, b = 0,02-0,04, c = 0,02-0,04, m = 3-9, n = 1-5

- Diméthicone de lauryl polyglycéryl-3 polyméthylsiloxyéthyle (formule (VI)) : 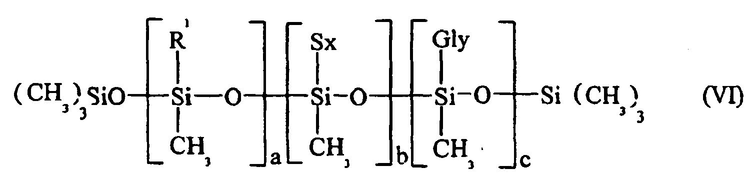 dans laquelle Sx, Gly, a, b, c, m et n ont la même signification que précédemment et R₁ est, soit un radical méthyle, soit un radical lauryle.
- Diméthicone de polyglycéryl-3 disiloxane (formule (VII)) : 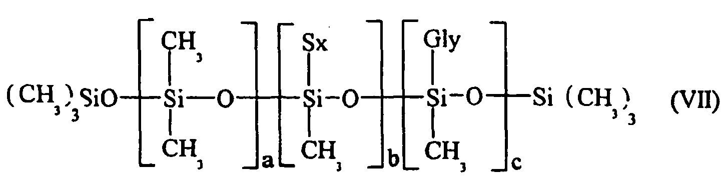
dans laquelle Gly, a, b, c, m et n ont la même signification que précédemment, et

Sx : -O(CH₃)₂SiO-Si(CH₃)₃

Les polymères de silicone de formule (l') sont décrits en détail dans la demande de brevet EP 1 213 316.

Selon un mode particulier de réalisation, le polymère de silicone de formule générale (I') est choisi parmi les polymères commercialisés par la société SHIN-ETSU sous les références KF6100, KF6104 et KF6105.

Le polymère commercialisé sous la référence KF6104 convient particulièrement à la préparation des compositions cosmétiques conformes à l'invention.

On peut également utiliser comme émulsionnant un organopolysioloxane réticulé élastomère émulsionnant à chaîne polyhydroxylée.

L' organopolysiloxane réticulé élastomère peut être obtenu par réaction d'addition réticulation de diorganopolysiloxane contenant au moins un hydrogène lié au silicium et de composés polyhydroxylés ou polyoxyalkylénés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁ -O-[Gly]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :
-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation , et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone émulsionnant peut être véhiculé sous forme de gel dans au moins une huile hydrocarbonée et/ou une huile siliconée. Dans ces gels, l'élastomère émulsionnant est souvent sous forme de particules non-sphériques.

Des élastomères polyglycérolés sont notamment décrits dans la demande WO-A-2004/024798 dont le contenu est incorporé dans la présente demande par référence.

Comme élastomère de silicone polyglycérolé, on peut utiliser ceux vendus sous les dénominations "KSG-710", "KSG-810", "KSG-820", "KSG-830", "KSG-840" par la société Shin Etsu.

Le tensioactif siliconé à chaîne polyhydroxylée peut être présent dans l'émulsion en une teneur allant de 0,1 % à 5 % en poids, par rapport au poids total de la composition, et de préférence allant de 1 % à 3 % en poids.

L'émulsion selon l'invention comprend aussi comme émulsionnant un alkyl C₈₋C₂₂ diméthicone copolyol, c'est-à-dire un poly méthyl alkyl(C₈-C₂₂) diméthyl méthyl siloxane oxypropyléné et/ou oxyéthyléné.

L'alkyl C₈-C₂₂ diméthicone copolyol est un composé de formule (I) suivante : 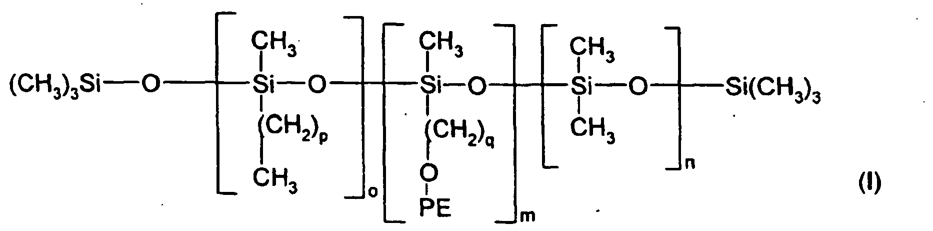 dans laquelle :
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanémént 0
- m allant de 1 à 40
- n allant de 10 à 200
- o allant de 1 à 100
- p allant de 7 et 21
- q allant de 0 à 4
et de préférence :
R=H
m = 1 à 10
n = 10 à 100
o = 1 à 30
p = 15
q=3

Comme alkyl C₈-C₂₂ diméthicone copolyol, on peut citer le cétyl diméthicone copolyol comme le produit commercialisé sous la dénomination Abil EM-90 par la société Goldschmidt.

L'alkyl C₈-C₂₂ diméthicone copolyol peut être présent dans l'émulsion en une teneur allant de 0,1 % à 5 % en poids, par rapport au poids total de la composition, et de préférence allant de 1 % à 3 % en poids.

L'émulsion peut comprendre un tensioactif additionnel différent de l'alkyl C₈-C₂₂ diméthicone copolyol décrit précédemment, notamment présent en une teneur pouvant aller de 0,05 % à 3 % en poids, par rapport au poids total de la composition, et de préférence allant de 0,1 % à 2 % en poids.

L'émulsion selon l'invention peut comprendre un tensioactif émulsionnant additionnel, différent indépendamment des tensioactifs siliconés à chaîne polyhydroxylée ou des alkyl C₈-C₂₂ diméthicone copolyol décrits précédemment, permettant l'obtention d'émulsion eau-dans-huile, notamment un tensioactif ayant un HLB (balance hydrophile/lipophile) inférieur à 7 ; un tel tensioactif émulsionnant peut être choisi parmi les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone ; les polymères du type ester d'acide gras de glycol polyoxyalkyléné, les tensioactifs siliconés à chaîne polyhydroxylée.

Comme autre tensioactif additionnel utilisable dans l'invention pour l'obtention d'une émulsion E/H on peut citer les polymères du type ester d'acide gras de glycol polyoxyalkyléné ayant des propriétés émulsionnantes eau-dans-huile.

L'ester d'acide gras dudit polymère est de préférence polyhydroxylé. En particulier, ce polymère est un polymère séquencé, de préférence de structure ABA, comportant des séquences poly(ester hydroxylé) et des séquences polyéthylèneglycols.

L'ester d'acide gras dudit polymère émulsionnant tel que défini ci-dessus présente en général une chaîne comportant de 12 à 20 atomes de carbone, de préférence de 14 à 18 atomes de carbone. Les esters peuvent notamment être choisis parmi les oléates, les palmitates ou les stéarates.

Les séquences polyéthylèneglycols dudit polymère émulsionnant tel que défini ci-dessus comportent de préférence de 4 à 50 moles d'oxyde d'éthylène, et de préférence encore de 20 à 40 moles d'oxyde d'éthylène.

Un tensioactif polymère convenant particulièrement à la réalisation des compositions de l'invention est le di-polyhydroxystéarate de polyéthylène glycol à 30 OE vendu sous la dénomination commerciale « Arlacel P 135 » par la société ICI.

L'émulsion peut comprendre un tensioactif additionnel différent du tensioactif siliconé à chaîne polyhydroxylée ou de l'alkyl C₈-C₂₂ diméthicone copolyol décrit précédemment, notamment présent en une teneur pouvant aller de 0,05 % à 3 % en poids, par rapport au poids total de la composition, et de préférence allant de 0,1 % à 2 % en poids.

La phase aqueuse de l'émulsion solide selon l'invention comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale. L'eau peut être présente dans l'émulsion selon l'invention en une teneur allant de 10 % à 40 % en poids, par rapport au poids total de la composition, de préférence allant de 15 % à 30 % en poids, et préférentiellement allant de 15 % à 25 % en poids.

La phase aqueuse peut également comprendre des solvants miscibles à l'eau (à température ambiante - 25 °C) comme par exemple les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbone, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ;
et leurs mélanges.

L'émulsion selon l'invention peut comprendre un solvant organique miscible à l'eau, notamment un polyol, en une teneur allant de 1 % à 20 % en poids, par rapport au poids total de la composition, et de préférence allant de 3 % à 15 % en poids.

La phase aqueuse peut comprendre en outre des agents de stabilisation , par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse peut être présente dans l'émulsion selon l'invention en une teneur allant 25 % à 50 %, en poids, de préférence allant de 25 % à 45 % en poids, par rapport au poids total de l'émulsion, et préférentiellement allant de 25 % à 35 % en poids.

La phase grasse de l'émulsion peut comprendre en outre au moins un corps gras pâteux.

Par "pâteux" au sens de la présente invention, on entend désigner un composé gras lipophile, à changement d'état solide/liquide réversible présentant à l'état solide une organisation cristalline anisotrope, et comportant à la température de 23°C une fraction liquide et une fraction solide..

Par composé pâteux au sens de l'invention, on entend un composé ayant une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline.

Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi :
- les composés siliconés polymères ou non,
- les composés fluorés polymères ou non,
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
   et leurs mélanges.

Le composé pâteux est de préférence polymère, notamment hydrocarboné.

### Composés pâteux siliconés et /ou fluorés

Un composé pâteux siliconé et fluoré préféré est le polyméthyl trifluoropropryl méthylalkyl diméthylsiloxane, fabriqué sous la dénomination X22-1088 par SHIN ETSU.

### Composés pâteux polyéthers

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique; et leurs mélanges.

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanôique, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux de monoacides carboxyliques aliphatiques mono hydroxylés linéaires, saturés ;
b) les esters partiels ou totaux de monoacides carboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé,
   et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Comme corps gras pâteux, on utilise de préférence un copolymère séquencé d'oxyde d'éthylène et/ou d'oxyde de propylène et d'oxyde d'alkylène ayant de 6 à 40 atomes de carbones, le copolymère ayant un poids moléculaire moyen en poids allant de 5000 à 8000.

Avantageusement, l'oxyde d'alkylène du copolymère peut comprendre de 6 à 30 atomes de carbone, de préférence de 8 à 20 atomes de carbone, préférentiellement de 10 à 18 atomes de carbone, et plus préférentiellement de 10 à 14 atomes de carbone.

Le poids moléculaire moyen en poids du copolymère va de 5000 à 8000, de préférence de 5500 à 7000, préférentiellement de 5500 à 6500, et plus préférentiellement de 5800 à 6200.

Le copolymère comprend avantageusement de 35 à 55 motifs d'oxyde d'éthylène et/ou d'oxyde de propylène et de 15 à 30 motifs d'oxyde d'alkylène ayant de 6 à 40 atomes de carbones.

En particulier, le copolymère est tel que le rapport entre le nombre de motifs d'oxyde d'éthylène et/ou d'oxyde de propylène et le nombre de motifs d'oxyde d'alkylène ayant de 6 à 40 atomes de carbones peut aller de 1,5 à 2,5, de préférence aller de 1,8 à 2,3 et préférentiellement aller de 1,9 à 2,1.

De tels copolymères sont notamment décrits dans le document FR-A-2425848 et vendus sous la dénomination "ELFACOS® ST 9" par la société AKZO NOBEL.

Le corps gras pâteux peut être présent dans la composition selon l'invention en une teneur allant de 1 % à 25 % en poids, par rapport au poids total de la composition, de préférence allant de 1 % à 18 % en poids, et préférentiellement allant de 1 % à 10 % en poids.

Avantageusement, l'émulsion solide selon l'invention a une dureté après au moins un mois à 37 °C telle que la force de pénétration soit supérieure à 45 g, notamment va de 45 g à 150 g.
On entend par dureté après au moins un mois à 37 °C la dureté mesurée après stockage de l'émulsion solide pendant au moins un mois à 37 °C, et notamment un mois à compter de la fin la préparation de l'émulsion solide.

En particulier, l'émulsion solide a une dureté telle que la force de pénétration après 24 heures à 20 °C (notamment à compter de la fin de la préparation de l'émulsion solide) et au moins 1 mois à 37 °C est supérieure ou égale à 45 grammes (g), notamment va de 45 g à 150 g.

De préférence, la dureté de l'émulsion solide va de 50 g à 130 g, préférentiellement va de 60 g à 130 g, et plus préférentiellement va de 70 g à 130 g.

La dureté de l'émulsion est mesurée selon le protocole suivant :

En fin de préparation de l'émulsion, cette dernière est coulée à chaud dans une coupelle et est maintenue 24 heures à 20 °C. On mesure alors sur l'émulsion solide la force de pénétration à l'aide d'un texturomètre vendu sous la dénomination TA-XT2i par la société RHEO, équipé du mobile de mesure TA24, diamètre de 4 mm, de la société STEVENS, en utilisant les conditions de mesure suivantes :
Force de déclenchement (trigger) = 1,0 g
Pré-vitesse = 2,0 mm/s
Vitesse de pénétration = 0,5 mm/s
Profondeur de pénétration = 2 mm

La force de pénétration exprimée en grammes se lit sur l'appareil.

On stocke ensuite l'émulsion solide pendant 1 mois à 37 °C puis on place l'émulsion pendant 24 heures à 20 °C avant d'effectuer la mesure de la dureté (dite dureté après 1 mois à 37 °C) selon les mêmes conditions décrites précédemment.

L'émulsion selon l'invention peut comprendre au moins une charge.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (NylonⓇ) , de poly-β-alanine et de polyéthylène, les poudres de polymères de tétrafluoroéthylène (TéflonⓇ), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes dans la composition en une teneur allant de 0,1 % à 25 % en poids, par rapport au poids total de la composition, de préférence allant de 1 % à 20 % en poids, et préférentiellement allant de 5 % à 15 % en poids.

Selon un mode de réalisation de l'invention, l'émulsion peut comprendre des particules de polyméthacrylate de méthyle. En particulier, ces particules ne sont pas filmogènes, c'est-à-dire qu'elles ne forment pas un film continu lorsqu'elle sont déposées sur un support tel que la peau.

Les poudres de polyméthacrylate de méthyle se présentent généralement sous la forme de particules sphériques creuses ou pleines de couleur blanche dont la taille moyenne en nombre est généralement à l'échelle du micromètre, en particulier varie de 3 à 15 microns et généralement varie de 3 à 10 microns. Par « taille moyenne en nombre », on désigne la dimension donnée par la distribution granulométrique statistique à la moitié de la population, dite D50.

Il est également possible de caractériser ces particules de polyméthacrylate de méthyle par leur densité, celle-ci étant susceptible de varier notamment en fonction de la taille de la cavité sphérique desdites particules.
Dans le cadre de la présente invention, cette densité est appréciée selon le protocole suivant, dit de la densité tassée :
On verse m=40 g de poudre dans une éprouvette graduée ; puis on place l'éprouvette sur l'appareil STAV 2003 de chez STAMPF VOLUMETER ; l'éprouvette est ensuite soumise à 1500 tassements ; puis on mesure directement sur l'éprouvette le volume final Vf de poudre tassée. La densité tassée est déterminée par le rapport m/Vf, en l'occurrence 40/Vf (Vf étant exprimé en cm³ et m en g).
En particulier, la densité des particules de polyméthacrylate de méthyle utilisables selon l'invention peut varier de 0,3 à 1,5 , notamment de 0,5 à 1,5 , et plus particulièrement de 1 à 1,5.

A titre représentatif et non limitatif de polyméthacrylate de méthyle convenant à l'invention, on peut notamment citer les particules de polyméthyméthacrylate commercialisées par la société MATSUMOTO YUSHI CO sous la dénomination « Micropearl M100 », par la société LCW sous la dénomination « Covabead LH 85 » et celles commercialisées par la société NIHON JUNYAKU sous la dénomination « JURYMER MB1 ».

Les particules de polyméthacrylate de méthyle peuvent être présentes en une teneur allant de 0,5 % à 10 % en poids, par rapport au poids total de la composition, de préférence allant de 0,5 % à 7 % en poids, préférentiellement allant de 1 % à 20 % en poids, et plus préférentiellement allant de 0,5 % à 5 % en poids.

L'émulsion selon l'invention peut comprendre au moins une matière colorante pouvant être choisie parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la composition.

Par « nacres », il faut comprendre des particules irisées, notamment produites par certains mollusques dans leur coquille ou bien synthétisées, insolubles dans le milieu de la composition.
Par « colorants », il faut comprendre des composés généralement organiques solubles dans les corps gras comme les huiles ou dans une phase hydroalcoolique.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.
On utilise de préférence des pigments d'oxydes de fer ou de dioxyde de titane.

Avantageusement, les pigments sont traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.
Les colorants hydrosolubles sont par exemple le jus de betterave, le bleu de méthylène, le caramel.

Les matières colorantes peuvent être présentes en une teneur allant de 0,5 % à 30 % en poids, par rapport au poids total de la composition, de préférence allant de 3 % à 20 % en poids, et préférentiellement allant de 5 à 15 % en poids.

Selon un mode particulier de réalisation de l'invention, l'émulsion peut comprendre au moins une matière pulvérulente enrobée hydrophobe, notamment en une teneur inférieure ou égale à 20 % en poids, par rapport au poids total de la composition. Ladite matière pulvérulente peut être choisie parmi les charges, les matières colorantes pulvérulentes comme les charges, les pigments tel que décrites précédemment. Ladite matière pulvérulente est enrobée avec un agent hydrophobe tel que décrit précédemment.

La composition peut contenir d'autres ingrédients cosmétiques usuels pouvant être choisis notamment parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut être préparé selon le mode opératoire suivant :
On prépare d'une part le mélange des constituants de la phase huileuse en mélangeant et en chauffant à une température comprise entre 70 °C et 120 °C les cires, le copolymère séquencé d'oxyde d'éthylène et/ou d'oxyde de propylène et d'oxyde d'alkylène ayant de 6 à 40 atomes de carbones, les huiles non volatiles, puis en ajoutant sous agitation, à une température comprise entre 60 °C et 80 °C, les huiles volatiles, les charges et les pigments.

On prépare d'autre part le mélange des constituants de la phase aqueuse comprenant l'eau, les tensioactifs, et les solvants miscibles à l'eau en chauffant à une température comprise entre 60 °C et 80 °C.

Puis on ajoute la phase aqueuse dans la phase huileuse, à une température comprise entre 60 ° et 80 °C, et on agite à l'aide d'une turbine jusqu'à l'obtention de l'émulsion eau-dans-huile. L'émulsion est ensuite coulée dans un contenant, par exemple une coupelle, puis refroidie à température ambiante jusqu'à l'obtention de l'émulsion solide.

L'invention est illustrée plus en détails par l'exemple décrit ci-après.

### Exemple 1 :

On a préparé un fond de teint solide ayant la composition suivante :

**Phase huileuse :**

| | |
|---|---|
| Cire de candelilla | 1,8 g |
| Ozokérite | 1,2 g |
| Isononanoate d'isotridécyle | 9,0 g |
| Cyclopentasiloxane | 22,5 g |
| Oxydes de fer enrobés par sel disodique de stéaroyl glutamate et d'hydroxyle d'aluminium | 2,2 g |
| Dioxyde de titane enrobés par sel disodique de stéaroyl glutamate et d'hydroxyle d'aluminium | 7,8 g |
| Silice pyrogénée hydrophobe (Aérosil R972 de chez Degussa) | 0,5 g |
| Microbilles de silice | 9,0 g |
| Polyméthacrylate de méthyle | 3 g |
| Nanopigments de dioxyde de titane | 3 g |
| Copolymère d'oxyde d'éthylène (45 OE) et d'époxydodécane (22 moles) vendu sous la dénomination "ELFACOS® ST 9" par la société AKZO NOBEL | 4 g |
| Isostéarate de polyglycéryle-4 | 1,50 g |
| Silicone à chaîne polyglycérolée (KF6104 de Shin Etsu) | 2 g |

**Phase aqueuse :**

| | |
|---|---|
| Eau | 20 g |
| Butylène glycol | 3 g |
| Glycérine | 5 g |
| Sulfate de magnésium | 1 g |
| Mélange de polydiméthylsiloxane oxyéthyléné oxypropyléné (18 OE/18 OP), de cyclopentasiloxane et d'eau (10/88/2) (DC 2-5225 C de DOW CORNING) | 2 g |
| Conservateurs | 1,50 g |

La composition a été préparée selon le mode opératoire suivant :
On chauffe à 90 °C le mélange comprenant les cires, le copolymère ELFACOS ST 9 et les huiles non volatiles jusqu'à obtention d'un mélange homogène et limpide ; puis on ajoute sous agitation à 70 °C les huiles volatiles, les pigments et les charges. On prépare ensuite le mélange des ingrédients de la phase aqueuse en chauffant à 70 °C, puis la phase aqueuse est introduite à 70 °C dans la phase huileuse, sous agitation jusqu'à obtention de l'émulsion eau-dans-huile.

L'émulsion est coulée à 70 °C dans une coupelle préalablement chauffée puis on laisse refroidir à la température ambiante jusqu'à obtention de l'émulsion solide.

On obtient un fond de teint solide ayant une dureté de 118,2 g mesurée à 24 heures à 20 °C et une dureté supérieure à 45 g mesurée après stockage à 37 °C pendant 1 mois, les mesures de dureté étant effectuées selon le protocole précédemment décrit.

Lors de l'application sur la peau, le fond de teint est crémeux et onctueux.

## Revendications

1. Emulsion solide eau-dans-huile comprenant une phase aqueuse émulsionnée dans une phase grasse comprenant une huile ester et une cire de Candelilla, et d'ozokérite, un tensioactif siliconé à chaîne polyhydroxylée, et un tensioactif additionnel alkyl C₈-C₂₂ diméthicone copolyol de formule (I) suivante : 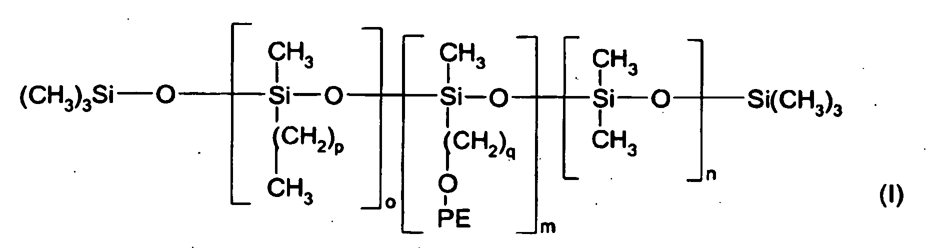 dans laquelle :
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanémént 0
- m allant de 1 à 40
- n allant de 10 à 200
- o allant de 1 à 100
- p allant de 7 et 21
- q allant de 0 à 4.

2. Emulsion selon la revendication précédente, **caractérisée par le fait que** la dureté va de 45 g à 150 g, de préférence va de 50 g à 130 g, préférentiellement va de 60 g à 130 g, et plus préférentiellement va de 70 g à 130 g.

3. Emulsion selon l'une quelconque des revendications précédentes, **caractérisée par le fait que** l'huile ester est un monoester.

4. Emulsion selon l'une quelconque des revendications précédentes, **caractérisée par le fait que** l'huile ester est un monoester de formule R₁COOR₂ dans laquelle R₁ représente une chaîne hydrocarbonée, linéaire ou ramifiée, comportant de 4 à 40 atomes de carbone, de préférence de 4 à 30 atomes de carbone, et préférentiellement de 7 à 20 atomes de carbone, et R₂ représente une chaîne hydrocarbonée ramifiée contenant de 3 à 40 atomes de carbone, de préférence de 10 à 30 atomes de carbone, et préférentiellement de 16 à 26 atomes de carbone.

5. Emulsion selon l'une quelconque des revendications précédentes, **caractérisée par le fait que** l'huile ester est choisie parmi le néopentanoate d'isodécyle, l'octanoate d'isocétyle, l'isononanoate d'isononyle, l'isononanoate d'isodécyle, l'isononanoate de tridécyle, le laurate d'hexyle, le laurate de 2-hexyl-décyle, le myristate d'isopropyle, le myristate d'isocétyle, le myristate d'isotridécyle le myristate de 2-octyldodécyle, le palmitate, d'isopropyle, le palmitate de 2-éthyl-hexyle, le palmitate d'isooctyle, le palmitate d'isocétyle, le palmitate d'isodécyle, le palmitate d'isostéaryle, le palmitate de 2-octyl-décyle, l'isostéarate d'isopropyle, le stéarate d'octyl-2 dodécyle, l'isostéarate d'isostéaryle, l'érucate d'octyl-2 dodécyle ;et leurs mélanges.

6. Emulsion selon l'une quelconque des revendications précédentes, **caractérisée par le fait que** l'huile ester est présente en une teneur allant de 5 % à 20 % en poids, par rapport au poids total de la composition, de préférence allant de 7 % à 15 % en poids, et préférentiellement allant de 8 % à 12 % en poids.

7. Emulsion selon l'une quelconque des revendications précédentes, **caractérisée par le fait que** la cire a une dureté allant de 5 MPa à 9 MPa.

8. Emulsion selon l'une quelconque des revendications précédentes, **caractérisée par le fait que** la cire a une dureté allant-de 6 MPa à 9 MPa, et de préférence allant de 7 MPa à 9 MPa.

9. Emulsion selon l'une quelconque des revendications précédentes, **caractérisée par le fait que** la cire est présente en une teneur allant de 1 % à 10 % en poids, par rapport au poids total de la composition, de préférence allant de 2 % à 7 % en poids, et préférentiellement allant de 2 % à 5 % en poids.

10. Emulsion selon l'une quelconque des revendications précédentes, **caractérisée par le fait que** le tensioactif siliconé à chaîne polyhydroxylée est un polymère de silicone représenté par la formule générale (I') suivante :
R¹ₐ R²_{b} R³_{c} SiO_{(4-a-b-c)/2} (I')
dans laquelle :
a) a, b et c sont tels que a varie de 1 à 2,5 ; et b et c, indépendamment l'un de l'autre, varient de 0.001 à 1,5.
b) R¹, identique ou différent, est choisi parmi :
- les radicaux alkyle en C₁ à C₃₀, le cas échéant substitués par un ou plusieurs atomes de fluor, groupements amino et/ou carboxy,
- les radicaux aryle, aralkyle, et
- les radicaux de formule générale (II) :
-C_{d}H_{2d}-O-(C₂H₄O)₆(C₃H₆O)_{f}R⁴ (II)
dans laquelle :
- R⁴ étant un radical hydrocarboné en C₄ à C₃₀, ou un radical R⁵-(CO)- avec R⁵ étant un radical hydrocarboné en C₁ à C₃₀, et
- d, e et f étant des entiers tels que d varie de 0 à 15, e et f, indépendamment l'un de l'autre, varient de 0 à 50, et
- leurs combinaisons.
c) R² est réprésenté par la formule générale suivante (III) :
-Q-O-X (III)
dans laquelle :
- Q est un radical hydrocarboné bivalent en C₂ à C₂₀, pouvant inclure au moins une liaison éther et/ou au moins une liaison ester, et
- X est un radical hydrocarboné polyhydroxylé.
d) R³ est un groupement organosiloxane de formule générale (IV) : 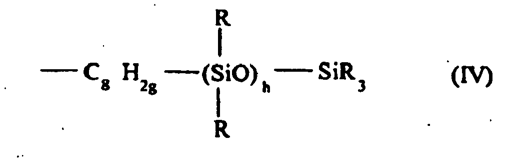 avec :
- chacun des radicaux R représentant, indépendamment l'un de l'autre, un radical choisi parmi les radicaux alkyle en C₁ à C₃₀, le cas échéant substitué par un ou plusieurs atomes de fluor, et les radicaux aryle et aralkyle,
- g et h étant des entiers tels que g varie de 1 à 5 et h varie de 0 à 500.

11. Emulsion selon la revendication précédente, **caractérisée par le fait que** le polymère de silicone est un composé de formule générale (I') dans laquelle R¹ est un radical alkyle en C₁ à C₁₀, en particulier de C₁ à C₆, et en particulier en C₁ à C₄.

12. Emulsion selon la revendication 10 ou 11, **caractérisée par le fait que** le polymère de silicone est un composé de formule générale (I') dans laquelle :
- a varie de 1 à 1,4, b et c, indépendamment l'un de l'autre, varient de 0,02 à 0,04, et
- R¹ est un radical alkyle en C₁ à C₁₀, en particulier de C₁ à C₆, et en particulier en C₁ à C₄,
- R² est représenté par la formule (IIIA) :
-C₃H₆O[CH₂CH(OH)CH₂O]ₙH (IIIA)
dans laquelle n varie de 1 à 5,
- R₃ est représenté par la formule (IVA) :
-C₂H₄(CH₃)₂SiO[(CH₃)₂SiO]ₘSi(CH₃)₂ (IVA)
dans laquelle m varie de 3 à 9.

13. Emulsion selon la revendication précédente, **caractérisée par le fait que** le polymère de silicone est un composé de formule générale (I') dans laquelle :
- a varie de 1 à 1,4, b et c, indépendamment l'un de l'autre, varient de 0,02 à 0,04, et
- R¹ est un radical méthyle,
- R² est représenté par la formule (IIIA), dans laquelle n varie de 1 à 5, et
- R³ est représenté par la formule (IVA) dans laquelle m varie de 3 à 9.

14. Emulsion selon l'une quelconque des revendications 1 à 13, **caractérisée par le fait que** le tensioactif siliconé à chaîne polyhydroxylée est un organopolysiloxane réticulé élastomère émulsionnant à chaîne polyhydroxylée, notamment à chaîne polyglycérolée.

15. Emulsion selon la revendication précédente, **caractérisée par le fait que** l'organopolysiloxane réticulé élastomère est obtenu par réaction d'addition réticulation de diorganopolysiloxane contenant au moins un hydrogène lié au silicium et de composés polyhydroxylés ayant des groupements à insaturation éthylénique, notamment en présence de catalyseur platine.

16. Emulsion selon la revendication 14 ou 15, **caractérisée par le fait que** l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

17. Emulsion selon la revendication précédente, **caractérisée par le fait que** le composé (B) est un composé polyglycérolé répondant à la formule (B') suivante :
CₘH₂ₘ₋₁-O-[Gly ]n-CₘH₂ₘ₋₁ (B')
dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :
-CH₂-CH(OH)-CH₂-O- ou -CH₂-CH(CH₂OH)-O-

18. Emulsion selon l'une quelconque des revendications précédentes, **caractérisée par le fait que** le tensioactif siliconé à chaîne polyhydroxylée est présent en une teneur allant de 0,1 % à 5 % en poids, par rapport au poids total de la composition, et de préférence allant de 1 % à 3 % en poids.

19. Emulsion selon les revendications précédentes, **caractérisée par le fait qu'**elle comprend un tensioactif émulsionnant additionnel choisi parmi les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone ; les polymères du type ester d'acide gras de glycol polyoxyalkyléné, et leurs mélanges.

20. Emulsion selon l'une quelconque des revendications précédentes, **caractérisée par le fait que** le tensioactif additionnel est choisi parmi les ester d'acides gras de polyols comme les mono-, di-, tri- ou sesqui-oléates ou stéarates de sorbitol ou de glycérol, les laurates de glycérol ou de polyéthylène glycol ; les polymères du type ester d'acide gras de glycol polyoxyalkyléné, les silicones polyhydroxylées, notamment polyglycérolées, les élastomères de silicone émulsionnants, les tensioactifs siliconés à chaîne polyhydroxyalkylénée.

21. Emulsion selon l'une quelconque des revendications 19 à 20, **caractérisée par le fait que** le tensioactif émulsionnant additionnel est présent en une teneur allant de 0,05 % à 3 % en poids, par rapport au poids total de la composition, et de préférence allant de 0,1 % à 2 % en poids.

22. Emulsion selon l'une quelconque des revendications précédentes, **caractérisée par le fait que** la phase grasse comprend une huile de silicone volatile.

23. Emulsion selon la revendication précédente, **caractérisée par le fait que** l'huile de silcone volatile est choisie parmi l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

24. Emulsion selon la revendication 22 ou 23, **caractérisée par le fait que** l'huile de silicone volatile est présente en une teneur allant de 5 % à 40 % en poids, par rapport au poids total de la composition, de préférence allant de 10 % à 30 % en poids, et préférentiellement allant de 15 % à 30 % en poids.

25. Emulsion selon l'une quelconque des revendications précédentes, **caractérisée par le fait qu'**elle comprend une huile additionnelle, différente des huiles esters et des huiles de silicone volatiles.

26. Emulsion selon la revendication précédente, **caractérisée par le fait que** l'huile additionnelle est choisie parmi les huiles hydrocarbonées, les huiles fluorées, volatiles ou non volatiles.

27. Emulsion selon la revendication 25 ou 26, **caractérisée par le fait que** l'huile additionnelle est présente en une teneur allant de 0,1 % à 10 % en poids, par rapport au poids total de la composition, et de préférence allant de 0,1 % à 5 % en poids.

28. Emulsion selon l'une quelconque des revendications précédentes, **caractérisée par le fait qu'**elle contient de l'eau en une teneur allant de 10 % à 40 % en poids, par rapport au poids total de la composition, de préférence allant de 15 % à 30 % en poids, et préférentiellement allant de 15 % à 25 % en poids.

29. Emulsion selon l'une quelconque des revendications précédentes, **caractérisée par le fait qu'**elle comprend un solvant miscible à l'eau choisi parmi les monoalcools ayant de 2 à 6 atomes de carbone, les polyols ayant de 2 à 20 atomes de carbones, les éthers de glycol ayant de 3 à 16 atomes de carbone, et leurs mélanges.

30. Emulsion selon la revendication précédente, **caractérisée par le fait que** le solvant miscible à l'eau est choisi parmi la glycérine, le propylène glycol, le butylène glycol, le pentylène glycol, l'hexylène glycol, le dipropylène glycol, le diéthylène glycol, les alkyl(C₁-C₄)éther de mono, di- ou tripropylène glycol, les alkyl(C₁-C₄)éthers de mono, di- ou triéthylène glycol, l'éthanol, l'isopropanol, et leurs mélanges.

31. Emulsion selon la revendication 29 ou 30, **caractérisée par le fait que** le solvant organique miscible à l'eau est présent en une teneur allant de 1 % à 20 % en poids, par rapport au poids total de la composition, et de préférence allant de 3 % à 15 % en poids.

32. Emulsion selon l'une quelconque des revendications précédentes, **caractérisée par le fait qu'**elle comprend un corps gras pâteux.

33. Emulsion selon la revendication précédente, **caractérisée par le fait que** le corps gras pâteux est un copolymère séquencé d'oxyde d'éthylène et/ou d'oxyde de propylène et d'oxyde d'alkylène ayant de 6 à 40 atomes de carbones, le copolymère ayant un poids moléculaire moyen en poids allant de 5000 à 8000.

34. Emulsion selon l'une quelconque des revendications précédentes, **caractérisée par le fait qu'**elle comprend au moins une charge.

35. Emulsion selon la revendication précédente, **caractérisée par le fait que** la charge est choisie parmi le talc, le mica, la silice, le kaolin, les poudres de polyamide , les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone.

36. Emulsion selon la revendication 34 ou 35, **caractérisée par le fait que** les charges sont présentes en une teneur allant de 0,1 % à 25 % en poids, par rapport au poids total de la composition, de préférence allant de 1 % à 20 % en poids, et préférentiellement allant de 5 % à 15 % en poids.

37. Emulsion selon l'une quelconque des revendications précédentes, **caractérisée par le fait qu'**elle comprend des particules de polyméthacrylate de méthyle.

38. Emulsion selon la revendication précédente, **caractérisée par le fait que** les particules de polyméthacrylate de méthyle sont présentes en une teneur allant de 0,5 % à 10 % en poids, par rapport au poids total de la composition, de préférence allant de 0,5 % à 7 % en poids, préférentiellement allant de 1 % à 20 % en poids, et plus préférentiellement allant de 0,5 % à 5 % en poids.

39. Emulsion selon l'une quelconque des revendications précédentes, **caractérisée par le fait qu'**elle comprend au moins une matière colorante.

40. Emulsion selon l'une quelconque des revendications précédentes, **caractérisée par le fait qu'**elle comprend au moins une matière pulvérulente enrobée hydrophobe.

41. Emulsion selon la revendication précédente , **caractérisée par le fait que** la matière pulvérulente est choisi parmi les charges et les matières colorantes pulvérulentes.

42. Emulsion selon l'une quelconque des revendications 40 et 41, **caractérisée par le fait que** la matière pulvérulente est enrobée avec un agent hydrophobe choisi parmi les silicones, les acides gras, les savons métalliques, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés, les acides aminés N-acylés ou leurs sels, la lécithine, le trisostéaryle titanate d'isopropyle, et leurs mélanges.

43. Emulsion selon l'une quelconque des revendications 41 à 42, **caractérisée par le fait que** la matière pulvérulente enrobée hydrophobe est présente en une teneur inférieure ou égale à 20 % en poids, par rapport au poids total de la composition.

44. Emulsion selon l'une quelconque des revendications précédentes, **caractérisée par le fait qu'**elle comprend un ingrédient cosmétique choisi parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

45. Emulsion selon l'une quelconque des revendications précédentes, **caractérisée par le fait que** la composition est un fond de teint, un fard à paupières, un fard à joue, un produit anticemes, un produit de maquillage du corps, un rouge à lèvres, une base de soin pour la peau, une crème de soin ; une composition de soin pour les lèvres ; une composition de protection solaire, une composition autobronzante ; un déodorant.

46. Fond de teint comprenant une composition selon l'une quelconque des revendications 1 à 45.

47. Procédé cosmétique de maquillage ou de soin non thérapeutique de la peau comprenant l'application sur la peau d'une composition selon l'une quelconque des revendications précédentes.

## Claims

1. Solid water-in-oil emulsion comprising an aqueous phase emulsified in a fatty phase comprising an ester oil and a candelilla and ozokerite wax, a silicone surfactant containing a polyhydroxylated chain, and an additional surfactant which is a C₈-C₂₂ alkyl dimethicone copolyol of formula (I) below: 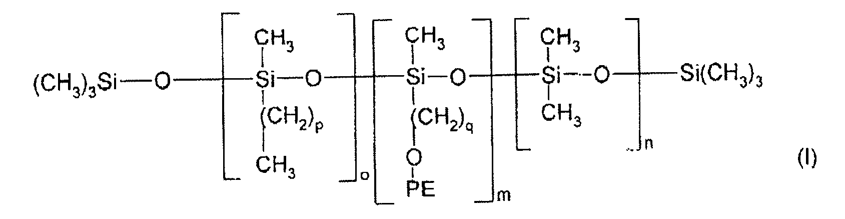 in which:
- PE represents (-C₂H₉O)ₓ-(C₃H₆O)_{y}-R, R being chosen from a hydrogen atom and an alkyl radical containing from 1 to 4 carbon atoms, x ranging from 0 to 100 and y ranging from 0 to 80, x and y not simultaneously being 0,
- m ranging from 1 to 40,
- n ranging from 10 to 200,
- o ranging from 1 to 100,
- p ranging from 7 to 21,
- q ranging from 0 to 4.

2. Emulsion according to the preceding claim,
**characterized in that** the hardness ranges from 45 g to 150 g, preferably from 50 g to 130 g, preferentially from 60 g to 130 g and more preferentially from 70 g to 130 g.

3. Emulsion according to either one of the preceding claims, **characterized in that** the ester oil is a monoester.

4. Emulsion according to any one of the preceding claims, **characterized in that** the ester oil is a monoester of formula R₁COOR₂ in which R₁ represents a linear or branched hydrocarbon-based chain containing from 4 to 40 carbon atoms, preferably from 4 to 30 carbon atoms and preferentially from 7 to 20 carbon atoms, and R₂ represents a branched hydrocarbon-based chain containing from 3 to 40 carbon atoms, preferably from 10 to 30 carbon atoms and preferentially from 16 to 26 carbon atoms.

5. Emulsion according to any one of the preceding claims, **characterized in that** the ester oil is chosen from isodecyl neopentanoate, isocetyl octanoate, isononyl isononanoate, isodecyl isononanoate, tridecyl isononanoate, hexyl laurate, 2-hexyldecyl laurate, isopropyl myristate, isocetyl myristate, isotridecyl myristate, 2-octyldodecyl myristate, isopropyl palmitate, 2-ethylhexyl palmitate, isooctyl palmitate, isocetyl palmitate, isodecyl palmitate, isostearyl palmitate, 2-octyldecyl palmitate, isopropyl isostearate, 2-octyldodecyl stearate, isostearyl isostearate and 2-octyldodecyl erucate, and mixtures thereof.

6. Emulsion according to any one of the preceding claims, **characterized in that** the ester oil is present in a content ranging from 5% to 20% by weight, preferably ranging from 7% to 15% by weight and preferentially ranging from 8% to 12% by weight relative to the total weight of the composition.

7. Emulsion according to any one of the preceding claims, **characterized in that** the wax has a hardness ranging from 5 MPa to 9 MPa.

8. Emulsion according to any one of the preceding claims, **characterized in that** the wax has a hardness ranging from 6 MPa to 9 MPa and preferably ranging from 7 MPa to 9 MPa.

9. Emulsion according to any one of the preceding claims, **characterized in that** the wax is present in a content ranging from 1% to 10% by weight, preferably ranging from 2% to 7% by weight and preferentially ranging from 2% to 5% by weight relative to the total weight of the composition.

10. Emulsion according to any one of the preceding claims, **characterized in that** the silicone surfactant containing a polyhydroxylated chain is a silicone polymer represented by the general formula (I') below:
R¹ₐR²_{b}R³_{c}SiO_{(4-a-b-c)/2} (I')
in which:
a) a, b and c are such that a ranges from 1 to 2.5; and b and c, independently of each other, range from 0.001 to 1.5,
b) R¹, which may be identical or different, is chosen from:
- C₁ to C₃₀ alkyl radicals, where appropriate substituted with one or more fluorine atoms and amino and/or carboxyl groups,
- aryl and aralkyl radicals, and
- the radicals of general formula (II):
-C_{d}H_{2d}-O-(C₂H₄)ₑ(C₃H₆O)_{f}R⁴ (II)
in which:
- R⁴ is a C₄ to C₃₀ hydrocarbon-based radical or a radical R⁵-(CO)- with R⁵ being a C₁ to C₃₀ hydrocarbon-based radical, and
- d, e and f are integers such that d ranges from 0 to 15, and e and f, independently of each other, range from 0 to 50, and
- combinations thereof,
c) R² is represented by the general formula (III) below:
-Q-O-X (III)
in which:
- Q is a divalent C₂ to C₂₀ hydrocarbon-based radical which may include at least one ether bond and/or at least one ester bond, and
- X is a polyhydroxylated hydrocarbon-based radical,
d) R³ is an organosiloxane group of general formula (IV): 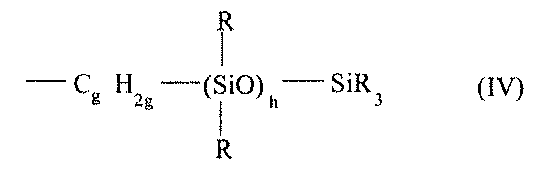 with:
- each of the radicals R representing, independently of each other, a radical chosen from C₁ to C₃₀ alkyl radicals, where appropriate substituted with one or more fluorine atoms, and aryl and aralkyl radicals,
- g and h being integers such that g ranges from 1 to 5 and h ranges from 0 to 500.

11. Emulsion according to the preceding claim, **characterized in that** the silicone polymer is a compound of general formula (I') in which R¹ is a C₁ to C₁₀, in particular a C₁ to C₆ and in particular C₁ to C₄ alkyl radical.

12. Emulsion according to Claim 10 or 11, **characterized in that** the silicone polymer is a compound of general formula (I') in which:
- a ranges from 1 to 1.4, b and c, independently of each other, range from 0.02 to 0.04, and
- R¹ is a C₁ to C₁₀, in particular a C₁ to C₆ and in particular a C₁ to C₄ alkyl radical,
- R² is represented by formula (IIIA):
-C₃H₆O[CH₂CH(OH)CH₂O]ₙH ( IIIA)
in which n ranges from 1 to 5, and
- R³ is represented by the formula (IVA):
-C₂H₄(CH₃)₂SiO[(CH₃)₂SiO]ₘSi(CH₃) (IVA)
in which m ranges from 3 to 9.

13. Emulsion according to the preceding claim, **characterized in that** the silicone polymer is a compound of general formula (I') in which:
- a ranges from 1 to 1.4, b and c, independently of each other, range from 0.02 to 0.04, and
- R¹ is a methyl radical,
- R² is represented by formula (IIIA) in which n ranges from 1 to 5, and
- R³ is represented by formula (IVA) in which m ranges from 3 to 9.

14. Emulsion according to any one of Claims 1 to 13, **characterized in that** the silicone surfactant containing a polyhydroxylated chain is an emulsifying elastomeric crosslinked organopolysiloxane containing a polyhydroxylated chain, especially a polyglycerolated chain.

15. Emulsion according to the preceding claim, **characterized in that** the elastomeric crosslinked organopolysiloxane is obtained by crosslinking addition reaction of diorganopolysiloxane containing at least one hydrogen linked to silicon and of polyhydroxylated compounds containing ethylenically unsaturated groups, especially in the presence of a platinum catalyst.

16. Emulsion according to Claim 14 or 15, **characterized in that** the elastomeric crosslinked organopolysiloxane is obtained by crosslinking addition reaction (A) of diorganopolysiloxane containing at least two hydrogens each linked to a silicon, and (B) of glycerolated compounds containing at least two ethylenically unsaturated groups, especially in the presence (C) of a platinum catalyst.

17. Emulsion according to the preceding claim, **characterized in that** compound (B) is a polyglycerolated compound corresponding to formula (B') below:
CₘH₂ₘ₋₁-O-[Gly ]n-CₘH₂ₘ₋₁ (B')
in which m is an integer ranging from 2 to 6, n is an integer ranging from 2 to 200, preferably ranging from 2 to 100, preferably ranging from 2 to 50, preferably n ranging from 2 to 20, preferably ranging from 2 to 10 and preferentially ranging from 2 to 5, and in particular equal to 3; Gly denotes:
-CH₂-CH(OH)-CH₂-O- or -CH₂-CH(CH₂OH)-O-.

18. Emulsion according to any one of the preceding claims, **characterized in that** the silicone surfactant containing a polyhydroxylated chain is present in a content ranging from 0.1% to 5% by weight and preferably ranging from 1% to 3% by weight relative to the total weight of the composition.

19. Emulsion according to the preceding claims, **characterized in that** it comprises an additional emulsifying surfactant chosen from fatty acid esters of polyols, for instance glyceryl or sorbitol mono-, di-, tri- or sesqui-oleates or -stearates, glyceryl or polyethylene glycol laurates; alkyl or alkoxy dimethicone copolyols containing an alkyl or alkoxy chain that is pendent or at the end of the silicone skeleton, for example containing from 6 to 22 carbon atoms; polymers such as polyoxyalkylenated fatty acid esters of glycol, and mixtures thereof.

20. Emulsion according to any one of the preceding claims, **characterized in that** the additional surfactant is chosen from fatty acid esters of polyols, for instance glyceryl or sorbitol mono-, di-, tri- or sesqui-oleates or -stearates, glyceryl or polyethylene glycol laurates; polymers such as polyoxyalkylenated fatty acid esters of glycol, polyhydroxylated and especially polyglycerolated silicones, emulsifying silicone elastomers, and silicone surfactants containing a polyhydroxylated chain.

21. Emulsion according to either one of Claims 19 and 20, **characterized in that** the additional emulsifying surfactant is present in a content ranging from 0.05% to 3% by weight and preferably ranging from 0.1% to 2% by weight relative to the total weight of the composition.

22. Emulsion according to any one of the preceding claims, **characterized in that** the fatty phase comprises a volatile silicone oil.

23. Emulsion according to the preceding claim, **characterized in that** the volatile silicone oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

24. Emulsion according to Claim 22 or 23, **characterized in that** the volatile silicone oil is present in a content ranging from 5% to 40% by weight, preferably ranging from 10% to 30% by weight and preferentially ranging from 15% to 30% by weight relative to the total weight of the composition.

25. Emulsion according to any one of the preceding claims, **characterized in that** it comprises an additional oil other than the ester oils and the volatile silicone oils.

26. Emulsion according to the preceding claim, **characterized in that** the additional oil is chosen from volatile or non-volatile hydrocarbon-based oils and fluoro oils.

27. Emulsion according to Claim 25 or 26, **characterized in that** the additional oil is present in a content ranging from 0.1% to 10% by weight and preferably ranging from 0.1% to 5% by weight relative to the total weight of the composition.

28. Emulsion according to any one of the preceding claims, **characterized in that** it contains water in a content ranging from 10% to 40% by weight, preferably ranging from 15% to 30% by weight and preferentially ranging from 15% to 25% by weight relative to the total weight of the composition.

29. Emulsion according to any one of the preceding claims, **characterized in that** it comprises a water-miscible solvent chosen from monoalcohols containing from 2 to 6 carbon atoms, polyols containing from 2 to 20 carbon atoms and glycol ethers containing from 3 to 16 carbon atoms, and mixtures thereof.

30. Emulsion according to the preceding claim, **characterized in that** the water-miscible solvent is chosen from glycerol, propylene glycol, butylene glycol, pentylene glycol, hexylene glycol, dipropylene glycol, diethylene glycol, mono-, di- or tripropylene glycol (C₁-C₄)alkyl ethers, mono-, di- or triethylene glycol (C₁-C₄)alkyl ethers, ethanol and isopropanol, and mixtures thereof.

31. Emulsion according to Claim 29 or 30, **characterized in that** the water-miscible organic solvent is present in a content ranging from 1% to 20% by weight and preferably ranging from 3% to 15% by weight relative to the total weight of the composition.

32. Emulsion according to any one of the preceding claims, **characterized in that** it comprises a pasty fatty substance.

33. Emulsion according to the preceding claim, **characterized in that** the pasty fatty substance is a block copolymer of ethylene oxide and/or propylene oxide and of an alkylene oxide containing from 6 to 40 carbon atoms, the copolymer having a weight-average molecular weight ranging from 5000 to 8000.

34. Emulsion according to any one of the preceding claims, **characterized in that** it comprises at least one filler.

35. Emulsion according to the preceding claim, **characterized in that** the filler is chosen from talc, mica, silica, kaolin, polyamide powders, poly-β-alanine powders, polyethylene powders, tetrafluoroethylene polymer powders, lauroyllysine, starch, boron nitride, hollow microspheres of polyvinylidene chloride/acrylonitrile, hollow microspheres of acrylic acid copolymers, silicone resin microbeads, polyorganosiloxane elastomer particles, precipitated calcium carbonate, magnesium carbonate and magnesium hydrocarbonate, hydroxyapatite, hollow silica microspheres, glass or ceramic microcapsules, and metal soaps derived from organic carboxylic acids containing from 8 to 22 carbon atoms.

36. Emulsion according to Claim 34 or 35, **characterized in that** the fillers are present in a content ranging from 0.1% to 25% by weight, preferably ranging from 1% to 20% by weight and preferentially ranging from 5% to 15% by weight relative to the total weight of the composition.

37. Emulsion according to any one of the preceding claims, **characterized in that** it comprises polymethyl methacrylate particles.

38. Emulsion according to the preceding claim, **characterized in that** the polymethyl methacrylate particles are present in a content ranging from 0.5% to 10% by weight, preferably ranging from 0.5% to 7% by weight, preferentially ranging from 1% to 20% by weight and more preferentially ranging from 0.5% to 5% by weight relative to the total weight of the composition.

39. Emulsion according to any one of the preceding claims, **characterized in that** it comprises at least one dyestuff.

40. Emulsion according to any one of the preceding claims, **characterized in that** it comprises at least one hydrophobic coated pulverulent material.

41. Emulsion according to the preceding claim, **characterized in that** the pulverulent material is chosen from pulverulent fillers and dyestuffs.

42. Emulsion according to either of Claims 40 and 41, **characterized in that** the pulverulent material is coated with a hydrophobic agent chosen from silicones, fatty acids, metal soaps, perfluoroalkyl phosphates, perfluoroalkyl silanes, perfluoroalkyl silazanes, polyhexafluoropropylene oxides, polyorganosiloxanes comprising perfluoroalkyl perfluoropolyether groups, amino acids, N-acylamino acids or salts thereof, lecithin and isopropyl triisostearyl titanate, and mixtures thereof.

43. Emulsion according to either of Claims 41 and 42, **characterized in that** the hydrophobic coated pulverulent material is present in a content of less than or equal to 20% by weight relative to the total weight of the composition.

44. Emulsion according to any one of the preceding claims, **characterized in that** it comprises a cosmetic ingredient chosen from hydrophilic or lipophilic gelling agents and/or thickeners, antioxidants, fragrances, preserving agents, neutralizers, sunscreens, vitamins, moisturizers, self-tanning compounds, anti-wrinkle active agents, emollients, hydrophilic or lipophilic active agents, free-radical scavengers, sequestering agents and film-forming agents, and mixtures thereof.

45. Emulsion according to any one of the preceding claims, **characterized in that** the composition is a foundation, an eyeshadow, a makeup rouge, a concealer product, a body makeup product, a lipstick, a skincare base, a care cream; a lipcare composition; an antisun composition, a self-tanning composition; a deodorant.

46. Foundation comprising a composition according to any one of Claims 1 to 45.

47. Non-therapeutic cosmetic process for making up or caring for the skin, comprising the application to the skin of a composition according to any one of the preceding claims.

## Patentansprüche

1. Feste Wasser-in-Öl Emulsion, die eine wässrige Phase enthält, die in einer Fettphase emulgiert ist, die einen Ester in Ölform, ein Candelillawachs und Ozokerit, einen siliconierten grenzflächenaktive Stoff mit polyhydroxylierter Kette und ein ergänzendes, grenzflächenaktives Alkyl(C₈₋₂₂)dimethiconcopolyol der folgenden Formel (I) enthält: 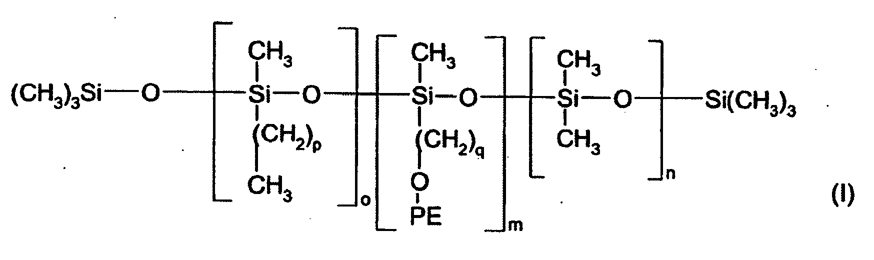 in der Formel:
· PE bedeutet (-C₂H₄O)ₓ-(C₃H₆O)_{y}-R, wobei R unter einem Wasserstoffatom und einer Alkylgruppe mit 1 bis 4 Kohlenstoffatomen ausgewählt ist, x im Bereich von 0 bis 100 und y im Bereich von 0 bis 80 liegt, wobei x und y nicht gleichzeitig Null bedeuten;
· m liegt im Bereich von 1 bis 40;
· n liegt im Bereich von 10 bis 200;
· o liegt im Bereich von 1 bis 100;
· p liegt im Bereich von 7 bis 21;
· q liegt im Bereich von 0 bis 4.

2. Emulsion nach dem vorhergehenden Ansprüch, **dadurch gekennzeichnet, dass** die Härte im Bereich von 45 bis 150 g, vorzugsweise 50 bis 130 g, noch bevorzugter 60 bis 130 g und besonders bevorzugt 70 bis 130 g liegt.

3. Emulsion nach einem der vorhergehenden Ansprüche, **dadurch gekennzeichnet, dass** es sich bei dem Ester in Ölform um einen Monoester handelt.

4. Emulsion nach einem der vorhergehenden Ansprüche, **dadurch gekennzeichnet, dass** der Ester in Ölform ein Monoester der Formel R₁COOR₂ ist, wobei R₁ eine lineare oder verzweigte Kohlenwasserstoffkette mit 4 bis 40 Kohlenstoffatomen, bevorzugt 4 bis 30 Kohlenstoffatomen und vorzugsweise 7 bis 20 Kohlenstoffatomen bedeutet und R₂ eine verzweigte Kohlenwasserstoffkette mit 3 bis 40 Kohlenstoffatomen, bevorzugt 10 bis 30 Kohlenstoffatomen und vorzugsweise 16 bis 26 Kohlenstoffatomen ist.

5. Emulsion nach einem der vorhergehenden Ansprüche, **dadurch gekennzeichnet, dass** der Ester in Ölform unter Isodecylneopentanoat, Isocetyloctanoat, Isononylisononanoat, Isodecylisononanoat, Tridecylisononanoat, Hexyllaurat, 2-Hexyldecyllaurat, Isopropylmyristat, Isocetylmyristat, Isotridecylmyristat, 2-Octyldodecylmyristat, Isopropylpalmitat, 2-Ethylhexylpalmitat, Isooctylpalmitat, Isocetylpalmitat, Isodecylpalmitat, Isostearylpalmitat, 2-Octyldecylpalmitat, Isopropylisostearat, 2-Octyldodecylstearat, Isostearylisostearat, 2-Octyldodecylerucat und deren Gemischen ausgewählt ist.

6. Emulsion nach einem der vorhergehenden Ansprüche, **dadurch gekennzeichnet, dass** der Ester in Ölform in einem Mengenanteil von 5 bis 20 Gew.-%, bezogen auf das Gesamtgewicht der Zusammensetzung, vorzugsweise 7 bis 15 Gew.-% und bevorzugt 8 bis 12 Gew.-% enthalten ist.

7. Emulsion nach einem der vorhergehenden Ansprüche, **dadurch gekennzeichnet, dass** das Wachs eine Härte von 5 bis 9 MPa aufweist.

8. Emulsion nach einem der vorhergehenden Ansprüche, **dadurch gekennzeichnet, dass** das Wachs eine Härte von 6 bis 9 MPa und vorzugsweise 7 bis 9 MPa aufweist.

9. Emulsion nach einem der vorhergehenden Ansprüche, **dadurch gekennzeichnet, dass** das Wachs in einem Mengenanteil von 1 bis 10 Gew.-%, bezogen auf das Gesamtgewicht der Zusammensetzung, vorzugsweise 2 bis 7 Gew.-% und noch bevorzugter 2 bis 5 Gew.-% vorliegt.

10. Emulsion nach einem der vorhergehenden Ansprüche, **dadurch gekennzeichnet, dass** der siliconierte grenzflächenaktive Stoff mit polyhydroxylierter Kette ein Siliconpolymer der folgenden allgemeinen Formel (I') ist:
R¹ₐ R²_{b} R³_{c} SiO(_{4-a-b-c)/2} (I')
in der Formel:
a) a, b und c sind so gewählt, dass a im Bereich von 1 bis 2,5 liegt; und b und c unabhängig voneinander im Bereich von 0,001 bis 1,5 liegen;
b) die Gruppen R¹, die gleich oder verschieden sind, sind ausgewählt unter:
- C₁₋₃₀-Alkylgruppen, die gegebenenfalls mit einem oder mehreren Fluoratomen, einer oder mehreren Amino- und/oder Carboxygruppen substituiert sind,
- Arylgruppen, Aralkylgruppen, und
- Gruppen der allgemeinen Formel (II):
-C_{d}H_{2d}-O-(C₂H₄O)ₑ(C₃H₆)_{f}R⁴ (II)
worin bedeuten:
- R⁴ eine C₄₋₃₀-Kohlenwasserstoffgruppe oder eine Gruppe R⁵-(CO)-, wobei R⁵ eine C₁₋₃₀-Kohlenwasserstoffgruppe ist, und
- d, e und f ganze Zahlen, die so gewählt sind, dass d im Bereich von 0 bis 15 liegt und e und f unabhängig voneinander im Bereich von 0 bis 50 liegen,
- und deren Kombinationen;
c) R² ist durch die allgemeine Formel (III) gegeben:
-Q-O-X (III)
worin bedeuten:
- Q eine zweiwertige C₂₋₂₀-Kohlenwasserstoffgruppe, die mindestens eine Etherbindung und/oder mindestens eine Esterbindung enthalten kann, und
- X eine polyhydroxylierte Kohlenwasserstoffgruppe;
d) R³ ist eine Organosiloxangruppe der allgemeinen Formel (IV): 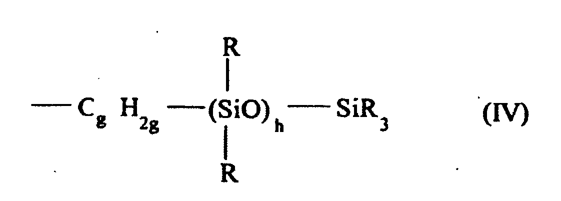 mit:
- jede der Gruppen R bedeutet unabhängig von den anderen eine Gruppe, die unter den C₁₋₃₀-Alkylgruppen, die gegebenenfalls mit einem oder mehreren Fluoratomen substituiert sind, Arylgruppen und Aralkylgruppen ausgewählt ist,
- wobei g und h ganze Zahlen sind, die so gewählt sind, dass g im Bereich von 1 bis 5 liegt und h im Bereich von 0 bis 500 liegt.

11. Emulsion nach dem vorhergehenden Anspruch, **dadurch gekennzeichnet, dass** das Siliconpolymer eine Verbindung der allgemeinen Formel (I') ist, worin R¹ eine Alkylgruppe mit 1 bis 10 Kohlenstoffatomen, insbesondere 1 bis 6 Kohlenstoffatomen und besonders 1 bis 4 Kohlenstoffatomen bedeutet.

12. Emulsion nach Anspruch 10 oder 11, **dadurch gekennzeichnet, dass** das Siliconpolymer eine Verbindung der allgemeinen Formel (I') ist, wobei:
- a im Bereich von 1 bis 1,4 liegt, b und c unabhängig voneinander im Bereich 0,02 bis 0,04 liegen,
- R¹ eine Alkylgruppe mit 1 bis 10 Kohlenstoffatomen, insbesondere 1 bis 6 Kohlenstoffatomen und besonders 1 bis 4 Kohlenstoffatomen bedeutet,
- R² durch die Formel (IIIA) gegeben ist:
-C₃H₆O[CH₂CH(OH)CH₂O]ₙH (IIIA)
worin n im Bereich von 1 bis 5 liegt,
- R³ durch die Formel (IVA) gegeben ist:
-C₂H₄(CH₃)₂SiO[(CH₃)₂SiO]ₘSi(CH₃)₂ (IVA)
worin m im Bereich von 3 bis 9 liegt.

13. Emulsion nach dem vorhergehenden Anspruch, **dadurch gekennzeichnet, dass** das Siliconpolymer eine Verbindung der allgemeinen Formel (I') ist, wobei:
- a im Bereich von 1 bis 1,4 liegt, b und c unabhängig voneinander im Bereich 0,02 bis 0,04 liegen,
- R¹ eine Methylgruppe bedeutet,
- R² durch die Formel (IIIA) gegeben ist, worin n im Bereich von 1 bis 5 liegt,
- R³ durch die Formel (IVA) gegeben ist, worin m im Bereich von 3 bis 9 liegt.

14. Emulsion nach einem der Ansprüche 1 bis 13, **dadurch gekennzeichnet, dass** der siliconierte grenzflächenaktive Stoff mit polyhydroxylierter Kette ein vernetztes elastomeres emulgierendes Organopolysiloxan mit polyhydroxylierter Kette und insbesondere mehrfach mit Glycerin veretherter Kette ist.

15. Emulsion nach dem vorhergehenden Anspruch, **dadurch gekennzeichnet, dass** das vernetzte elastomere Organopolysiloxan durch vernetzende Addition eines Diorganopolysiloxans, das mindestens ein an Silicium gebundenes Wasserstoffatom aufweist, und hydroxylierten Verbindungen, die Gruppen mit ethylenisch ungesättigter Bindung aufweisen, insbesondere in Gegenwart eines Platinkatalysators gebildet wird.

16. Emulsion nach Anspruch 14 oder 15, **dadurch gekennzeichnet, dass** das vernetzte elastomere Organopolysiloxan durch vernetzende Addition (A) eines Diorganopolysiloxans, das mindestens zwei jeweils an Silicium gebundene Wasserstoffatome aufweist, und (B) mit Glycerin veretherten Verbindungen, die mindestens zwei Gruppen mit ethylenisch ungesättigter Bindung aufweisen, insbesondere in Gegenwart (C) eines Platinkatalysators gebildet wird.

17. Emulsion nach dem vorhergehenden Anspruch, **dadurch gekennzeichnet, dass** die Verbindung (B) eine mehrfach mit Glycerin veretherte Verbindung ist, die der folgenden Formel (B') entspricht:
CₘH₂ₘ₋₁-O-[Gly]n-CₘH₂ₘ₋₁ (B')
wobei m eine ganze Zahl von 2 bis 6 ist, n eine ganze Zahl von 2 bis 200, vorzugsweise 2 bis 100 und bevorzugt 2 bis 50 bedeutet, wobei n vorzugsweise im Bereich von 2 bis 20, bevorzugt 2 bis 10 und noch bevorzugter 2 bis 5 liegt und insbesondere 3 ist; Gly bedeutet:
-CH₂-CH(OH)-CH₂-O- oder -CH₂-CH(CH₂OH)-O-.

18. Emulsion nach einem der vorhergehenden Ansprüche, **dadurch gekennzeichnet, dass** der siliconierte grenzflächenaktive Stoff mit polyhydroxylierter Kette in einem Mengenanteil von 0,1 bis 5 Gew.-%, bezogen auf das Gesamtgewicht der Zusammensetzung, und vorzugsweise 1 bis 3 Gew.-% enthalten ist.

19. Emulsion nach den vorhergehenden Ansprüchen, **dadurch gekennzeichnet, dass** sie einen ergänzenden emulgierenden grenzflächenaktiven Stoff enthält, der unter den Estern von Fettsäuren und Polyolen, wie Mono-, Di-, Tri- oder Sesquioleaten oder -stearaten von Sorbit oder Glycerin, Lauraten von Glycerin oder Polyethylenglycol; Alkyl- oder Alkoxydimethiconcopolyolen mit einer Alkyl- oder Alkoxygruppe als Seitenkette oder am Ende des Silicongerüsts, die beispielsweise 6 bis 22 Kohlenstoffatome aufweist; Polymeren vom Typ der polyalkoxylierten Glycolfettsäureester und deren Gemischen ausgewählt ist.

20. Emulsion nach einem der vorhergehenden Ansprüche, **dadurch gekennzeichnet, dass** der ergänzende grenzflächenaktive Stoff unter den Estern von Fettsäuren und Polyolen, wie Mono-, Di-, Tri- oder Sesquioleaten oder -stearaten von Sorbit oder Glycerin, Lauraten von Glycerin oder Polyethylenglycol; Polymeren vom Typ der polyalkoxylierten Glycolfettsäureester; polyhydroxylierten Siliconen und insbesondere mehrfach mit Glycerin veretherten Siliconen, emulgierenden Siliconelastomeren, siliconierten grenzflächenaktiven Stoffen mit Polyhydroxyalkylenkette ausgewählt ist.

21. Emulsion nach einem der Ansprüche 19 bis 20, **dadurch gekennzeichnet, dass** der ergänzende emulgierende grenzflächenaktive Stoff in einem Mengenanteil von 0,05 bis 3 Gew.-%, bezogen auf das Gesamtgewicht der Zusammensetzung, und vorzugsweise 0,1 bis 2 Gew.-% enthalten ist.

22. Emulsion nach einem der vorhergehenden Ansprüche, **dadurch gekennzeichnet, dass** die Fettphase ein flüchtiges Siliconöl enthält.

23. Emulsion nach dem vorhergehenden Anspruch, **dadurch gekennzeichnet, dass** das flüchtige Siliconöl unter Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemischen ausgewählt ist.

24. Emulsion nach Anspruch 22 oder 23, **dadurch gekennzeichnet, dass** das flüchtige Siliconöl in einem Mengenanteil von 5 bis 40 Gew.-%, bezogen auf das Gesamtgewicht der Zusammensetzung, vorzugsweise 10 bis 30 Gew.-% und noch bevorzugter 15 bis 30 Gew.-% vorliegt.

25. Emulsion nach einem der vorhergehenden Ansprüche, **dadurch gekennzeichnet, dass** sie ein ergänzendes Öl enthält, das von den Estern in Ölform und den flüchtigen Siliconölen verschieden ist.

26. Emulsion nach dem vorhergehenden Anspruch, **dadurch gekennzeichnet, dass** das ergänzende Öl unter den Kohlenwasserstoffölen, den fluorierten Ölen, die flüchtig oder nicht flüchtig sind, ausgewählt ist.

27. Emulsion nach Anspruch 25 oder 26, **dadurch gekennzeichnet, dass** das ergänzende Öl in einem Mengenanteil von 0,1 bis 10 Gew.-%, bezogen auf das Gesamtgewicht der Zusammensetzung, und vorzugsweise 0,1 bis 5 Gew.-% enthalten ist.

28. Emulsion nach einem der vorhergehenden Ansprüche, **dadurch gekennzeichnet, dass** sie Wasser in einem Mengenanteil von 10 bis 40 Gew.-%, bezogen auf das Gesamtgewicht der Zusammensetzung, bevorzugt 15 bis 30 Gew.-% und vorzugsweise 15 bis 25 Gew.-% enthält.

29. Emulsion nach einem der vorhergehenden Ansprüche, **dadurch gekennzeichnet, dass** sie ein mit Wasser mischbares Lösemittel enthält, das unter den Monoalkoholen mit 2 bis 6 Kohlenstoffatomen, Polyolen mit 2 bis 20 Kohlenstoffatomen, Glycolethern mit 3 bis 16 Kohlenstoffatomen und deren Gemischen ausgewählt ist.

30. Emulsion nach dem vorhergehenden Anspruch, **dadurch gekennzeichnet, dass** das mit Wasser mischbare Lösemittel unter Glycerin, Propylenglycol, Butylenglycol, Pentylenglycol, Hexylenglycol, Dipropylenglycol, Diethylenglycol, Alkyl(C₁₋₄)ethern von Mono-, Di- oder Tripropylenglycol, Alkyl(C₁₋₄)ethern von Mono-, Di- oder Triethylenglycol, Ethanol, Isopropanol und deren Gemischen ausgewählt ist.

31. Emulsion nach Anspruch 29 oder 30, **dadurch gekennzeichnet, dass** das mit Wasser mischbare organische Lösemittel in einem Mengenanteil von 1 bis 20 Gew.-%, bezogen auf das Gesamtgewicht der Zusammensetzung, und vorzugsweise 3 bis 15 Gew.-% enthalten ist.

32. Emulsion nach einem der vorhergehenden Ansprüche, **dadurch gekennzeichnet, dass** sie eine pastöse Fettsubstanz enthält.

33. Emulsion nach dem vorhergehenden Anspruch, **dadurch gekennzeichnet, dass** die pastöse Fettsubstanz ein Sequenzcopolymer von Ethylenoxid und/oder Propylenoxid und Alkylenoxid mit 6 bis 40 Kohlenstoffatomen ist, wobei das Copolymer eine gewichtsmittlere Molmasse von 5000 bis 8000 aufweist.

34. Emulsion nach einem der vorhergehenden Ansprüche, **dadurch gekennzeichnet, dass** sie mindestens eine Füllstoff enthält.

35. Emulsion nach dem vorhergehenden Anspruch, **dadurch gekennzeichnet, dass** der Füllstoff unter Talk, Glimmer, Kieselsäure, Kaolin, Polyamidpulvern, Poly-β-alaninpulvern, Polyethylenpulvern, Pulvern von Tetrafluorethylenpolymeren, Lauroyllysin, Stärke, Bornitrid, Mikrohohlkugeln aus Polyvinylidenchlorid/Acrylnitril, Mikrohohlkugeln aus Acrylsäurecopolymeren, Siliconharzmikrokugeln, Partikeln aus elastomeren Polyorganosiloxanen, gefälltem Calciumcarbonat, Magnesiumcarbonat und Magnesiumhydrogencarbonat, Hydroxyapatit, Siliciumdioxidmikrohohlkugeln, Mikrokapseln aus Glas oder Keramik, Metallseifen, die von organischen Carbonsäuren mit 8 bis 22 Kohlenstoffatomen abgeleitet sind, ausgewählt ist.

36. Emulsion nach Anspruch 34 oder 35, **dadurch gekennzeichnet, dass** die Füllstoffe in einem Mengenanteil von 0,1 bis 25 Gew.-%, bezogen auf das Gesamtgewicht der Zusammensetzung, bevorzugt 1 bis 20 Gew.-% und vorzugsweise 5 bis 15 Gew.-% enthalten sind.

37. Emulsion nach einem der vorhergehenden Ansprüche, **dadurch gekennzeichnet, dass** sie Partikel von Polymethylmethacrylat enthält.

38. Emulsion nach dem vorhergehenden Anspruch, **dadurch gekennzeichnet, dass** die Partikel von Polymethylmethacrylat in einem Mengenanteil von 0,5 bis 10 Gew.-%, bezogen auf das Gesamtgewicht der Zusammensetzung, bevorzugt 0,5 bis 7 Gew.-%, noch bevorzugter 1 bis 20 Gew.-% und vorzugsweise 0,5 bis 5 Gew.-% enthalten sind.

39. Emulsion nach einem der vorhergehenden Ansprüche**, dadurch gekennzeichnet, dass** sie mindestens ein Farbmittel enthält.

40. Emulsion nach einem der vorhergehenden Ansprüche, **dadurch gekennzeichnet, dass** sie mindestens eine pulverförmige hydrophobe umhüllte Substanz enthält.

41. Emulsion nach dem vorhergehenden Anspruch, **dadurch gekennzeichnet, dass** die pulverförmige Substanz unter den pulverförmigen Füllstoffen und Farbmitteln ausgewählt ist.

42. Emulsion nach einem der Ansprüche 40 und 41, **dadurch gekennzeichnet, dass** die pulverförmige Substanz mit einem hydrophoben Stoff umhüllt ist, der unter den Siliconen, Fettsäuren, Metallseifen, Perfluoralkylphosphaten, Perfluoralkylsilanen, Perfluoralkylsilazanen,Hexafluorpropylenpolyoxiden, Polyorganosiloxanen, die Perfluoralkylperfluorpolyethergruppen enthalten, Aminosäuren, N-acylierten Aminosäuren oder deren Salzen, Lecithin, Isopropyltriisostearyltitanat und deren Gemischen ausgewählt ist.

43. Emulsion nach einem der Ansprüche 41 bis 42, **dadurch gekennzeichnet, dass** die pulverförmige hydrophobe umhüllte Substanz in einem Mengenanteil von kleiner oder gleich 20 Gew.-%, bezogen auf das Gesamtgewicht der Zusammensetzung, enthalten ist.

44. Emulsion nach einem der vorhergehenden Ansprüche, **dadurch gekennzeichnet, dass** sie einen kosmetischen Bestandteil enthält, der unter den hydrophilen oder lipophilen Gelbildnern und/ oder Verdickungsmitteln, Antioxidantien, Parfums, Konservierungsmitteln, Neutralisationsmitteln, Sonnenschutzfiltern, Vitaminen, Hydratisierungsmitteln, Selbstbräunungsmitteln, Wirkstoffen gegen Falten, Emollientien, hydrophilen oder lipophilen Wirkstoffen, Radikalfängern für freie Radikale, Maskierungsmitteln, Filmbildnern und deren Gemischen ausgewählt ist.

45. Emulsion nach einem der vorhergehenden Ansprüche, **dadurch gekennzeichnet, dass** es sich bei der Zusammensetzung um ein Make-up, einen Lidschatten, ein Wangenrouge, ein Produkt gegen Augenringe, ein Produkt zum Schminken des Körpers, einen Lippenstift, eine pflegende Grundlage für die Haut, eine Pflegecreme; eine Zusammensetzung für die Pflege der Lippen; eine Zusammensetzung für den Sonnenschutz, ein Selbstbräunungsmittel; ein Deodorant handelt.

46. Make-up, das eine Zusammensetzung nach einem der Ansprüche 1 bis 45 umfasst.

47. Kosmetisches Verfahren zum Schminken oder für die nicht therapeutische Pflege der Haut, das das Aufbringen einer Zusammensetzung nach einem der vorhergehenden Ansprüche auf die Haut umfasst.
